(19) Europäisches Patentamt
European Patent Office
Office européen des brevets

(11) Veröffentlichungsnummer: **0 000 474**
**B1**

(12) # EUROPÄISCHE PATENTSCHRIFT

(21) Anmeldenummer: **78100218.3**

(22) Anmeldetag: **22.06.78**

(51) Int. Cl.³: **C 07 C 79/46,**
C 07 C 153/09,
A 01 N 37/10

(54) **Substituierte 3-(2'-Nitro-phenoxy)-alpha-phenoxy alkancarbonsäure und ihre Derivate, deren Herstellung und Verwendung als Herbizide**

(30) Priorität: **30.06.77 CH 8068/77**

(43) Veröffentlichungstag der Anmeldung:
**07.02.79 Patentblatt 79/03**

(45) Bekanntmachung des Hinweises auf die Patenterteilung:
**26.11.80 Patentblatt 80/24**

(84) Benannte Vertragsstaaten:
**BE CH DE FR GB NL**

(56) Entgegenhaltungen:
**DE - A - 2 639 796**
**FR - A - 2 325 638**
**FR - A - 2 348 182**
**NL - A - 77 07921**
**NL - A - 77 08091**

(73) Patentinhaber: **CIBA-GEIGY AG**
**Patentabteilung Postfach**
**CH - 4002 Basel (CH)**

(72) Erfinder: **Rohr, Otto, Dr.**
**Kilbertweg 19**
**CH - 4106 Therwil (CH)**
**Pissiotas, Georg, Dr.**
**Breslauer Strasse 8**
**D - 7850 Lörrach (DE)**
**Dürr, Dieter, Dr.**
**Brändelistalweg 16**
**CH - 4103 Bottmingen (CH)**
**Böhner, Beat, Dr.**
**Hügelweg 3**
**CH - 4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 474 B1

Substituierte 3-(2′-Nitro-phenoxy)-alpha-phenoxy-alkancarbonsäuren und ihre Derivate, deren Herstellung und Verwendung als Herbizide

Die Erfindung betrifft neue, herbizid und regulierend auf das Pflanzenwachstum einwirkende, substituierte 3-(2′-Nitro-phenoxy)-alpha-phenoxy-alkancarbonsäuren und ihre Derivate, Verfahren zu ihrer Herstellung, Mittel die diese Derivate als Wirkstoff enthalten sowie die Verwendung derselben oder sie enthaltender Mittel zur Bekämpfung von unerwünschtem Pflanzenwachstum oder zur Regulierung desselben.

Die substituierten 3-(2′-Nitro-phenoxy)-alpha-phenoxy-alkancarbonsäuren und ihre Derivate entsprechen der allgemeincy Formel I

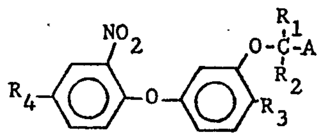

(I)

worin $R_1$ Wasserstoff, $C_1$—$C_4$-Alkyl oder $C_2$—$C_8$-Alkoxyalkyl,
$R_2$ Wasserstoff oder $C_1$—$C_4$-Alkyl,
$R_3$ Halogen, Cyano oder Nitro,
$R_4$ Halogen, Cyano oder Trifluormethyl,
A Cyan oder die Gruppe —COB,
B einen Rest $OR_5$, $SR_6$ oder $NR_7R_8$,
$R_5$ Wasserstoff oder

das Kation einer Base $\frac{1}{n} M^{n\oplus}$

wobei
M ein Alkali-, Erdalkalimetall-Kation oder einen Ammoniumrest

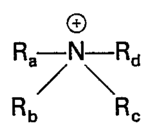

n als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, —$NH_2$ oder $C_1$—$C_4$-Alkoxy substituierten $C_1$—$C_4$-Alkylrest bedeuten,

$R_5$ bedeutet weiter einen $C_1$—$C_8$-Alkylrest, der unsubstituiert oder durch Halogen, Cyan, Nitro, Hydroxyl oder einen 5—6 gliedrigen Stickstoff enthaltenden Heterocyclus substituiert sein kann sowie einen $C_2$—$C_8$-Alkylrest, der durch Sauerstoff, Schwefel, Stickstoff, Carbonyl oder Carbonyloxy unterbrochen sein kann,
einen $C_3$—$C_6$-Cycloalkylrest,
einen $C_3$—$C_6$-Alkenyl- oder Alkinylrest, der durch Halogen substituiert sein kann,
einen Phenyl oder Benzylrest, der durch Halogen, Cyan, Nitro, $C_1$—$C_4$-Alkyl, $C_1$—$C_4$-Alkoxy oder Trifluormethyl substituiert sein kann,

$R_6$ bedeutet einen $C_1$—$C_8$-Alkylrest, der durch Halogen, Cyano, Nitro oder Hydroxyl substituiert, oder Sauerstoff, Schwefel, Stickstoff, Carbonyl oder Carbonyloxy unterbrochen sein kann,
einen $C_3$—$C_6$-Cycloalkylrest,
einen $C_3$—$C_6$-Alkenyl- oder Alkinylrest, der durch Halogen substituiert sein kann,
einen Phenyl- oder Benzylrest, der durch Halogen, Cyan, Nitro, $C_1$—$C_4$-Alkyl, $C_1$—$C_4$-Alkoxy oder Trifluormethyl substituiert sein kann,

$R_7$ Wasserstoff, einen $C_1$—$C_8$-Alkylrest, der durch Halogen, Cyan oder Hydroxyl substituiert sein kann, sowie einen $C_2$—$C_8$-Alkylrest, der durch Sauerstoff unterbrochen sein kann,

$R_8$ Wasserstoff, einen $C_1$—$C_4$-Alkylrest oder einen $C_3$—$C_4$-Alkylrest,

$R_7$ und $R_8$ zusammen mit dem Stickstoffatom an das sie gebunden sind, auch einen 5—6 gliedrigen heterocyclischen Rest, der durch Sauerstoff, Schwefel, eine Imino-, $C_1$—$C_4$-Alkylimino- oder Phenyliminogruppe unterbrochen sein kann, bedeuten.

In dieser allgemeinen Formel sind die Alkylreste sowohl verzweigt wie unverzweigt und enthalten die un gegebene Anzahl Kohlenstoffatome. Die Reste $R_7$ und $R_8$ sind bevorzugt Wasserstoff oder niedere Alkylgruppen. Einzelne unter ihnen können jedoch auch ungesättigt oder aromatisch (Phenyl) oder araliphatisch (Benzyl) sein und beide können zusammen mit dem Stickstoffatom an das sie gebunden sind, einen 5—6 gliedrigen Heterocyclus bilden.

Es sind herbizide Wirkstoffe auf der Basis von substituierten Diphenyläthern bekannt geworden, z.B. aus der schweizerischen Patentschrift No. 424 326. Neuerdings wurden herbizid wirksame Phenoxy-phenoxy-alkancarbonsäuren, siehe z.B. die DE - OS 2 433 067, beschrieben.

Diese Erfindung erschliesst eine neue Gruppe von Phenoxy-phenoxy-alkancarbonsäure-Derivaten, die in geringen Aufwandmengen das Wachstum der Pflanzen in einer für die Landwirtschaft vorteilhafter Weise zu beeinflussen vermögen.

Die neuen Wirkstoffe der allgemeinen Formel I gemäss Erfindung besitzen eine allgemeine, nicht spezifisch gegen Gräser gerichtete Herbizidwirkung, vor allem bei post-emergenter Anwendung und können in mono- und dicotylen, Kulturen als Unkrautmittel eingesetzt werden.

Die erfindungsgemässen Wirkstoffe besitzen auch günstige wachstumsregulierende Effekte (Wuchshemmung). Sie hemmen vor allem das Wachstum von dicotylen Pflanzen. Beispiele für die nutzbringende Anwendung der erfindungsgemässen Verbindungen sind z.B.

— die Reduktion des vegetativen Wachstums bei Soja und ähnlichen Leguminosen, was zu einer Ertragssteigerung dieser Kultur führt;
— die Hemmung des unerwünschten Wachstums von Geiztrieben bei Tabak, dessen Haupttrieb man geschnitten hat, was der Ausbildung grösserer und schönerer Blätter zugute kommt;
— die Hemmung des Wachstums von Gras und dicotyledonen Pflanzen, wie Obstbäume, Zierbäume, Gebüsche und Hecken, zwecks Einsparung an Schnittarbeit.

Die Verbindungen Erfindung sind wenig giftig für Warmblüter; deren Applikation dürfte keine Probleme verursachen. Die vorgeschlagenen Aufwandmengen liegen zwischen 0.1 und 5 kg pro Hektar.

Die Herstellung der neuen Verbindungen der allgemeinen Formel I erfolgt nach an sich bekannten Reaktionen z.B. gemäss dem Schema:

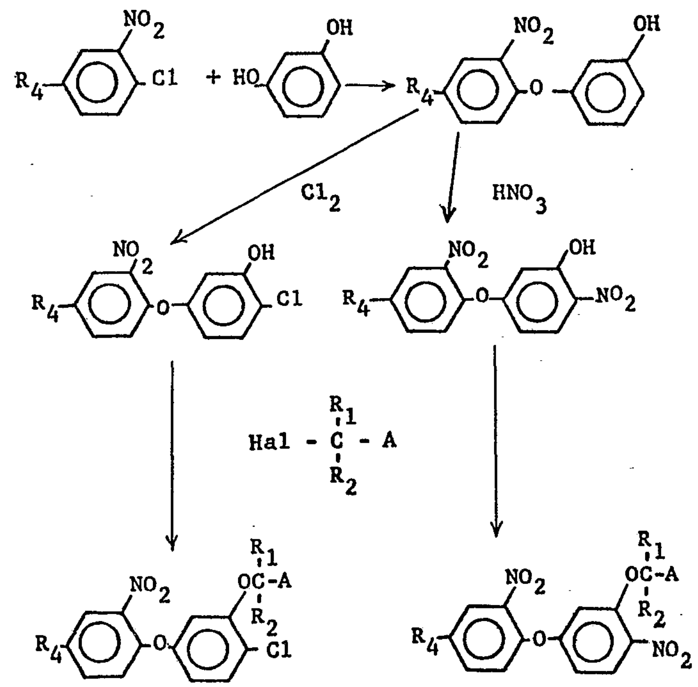


In diesen Formeln bedeutet Hal ein Halogenatom, vorzugsweise Chlor oder Brom, während A, $R_1$, $R_2$ und $R_4$ die in der allgemeinen Formel I an gegebene Bedeutung haben.

In para-Stellung durch den Rest $R_4$ substituiertes ortho Nitro-chlorbenzol wird in Gegenwart eines säurebindenden Mittels bei erhöhter Temperatur mit Resorcin umgesetet. Das Resorcin sollte gegenüber dem Chlorbenzol in leichtem Ueberschuss vorhanden sein. Nach vollzogener Reaktion wird die Reaktionsmasse in Wasser gegeben und der entstandene Diphenyläther mit einem mit Wasser nicht mischbaren Lösungsmittel aus der wässrigen Suspension extrahiert; zur Beschleunigung der Kondensation kann eine Spur Kupferpulver zugegeben werden. Die Kondensation wird vorteilhafterweise im Oelbad bei ca. 200°C durchgeführt, vgl: Weygand Hilgetag "Organisch Chemische Experimentierkunst, 3. Auflage 1964, 435; U.S. Patent No. 3 080 225, DE—OS 2 433 066 und DE—AS 1 187 852.

Durch Nitrieren in Eisessig, Aethylenchlorid oder Schwefelsäure kann in die noch freie para-Stellung des Diphenyläthers eine Nitrogruppe eingeführt werden. Durch Behandeln mit Chlor in Eisessig kann in diese Stellung ein Chloratom eingeführt werden. Hat $R_3$ die Bedeutung von Cyan oder Brom, so

wird in diesem Stadium das Chloratom z.B. durch Umsetzung mit KCN [s. Adv.Chem.Ser. *132*, (1974), 252 oder J.Organomet.Chem., *54* (1973)C, 57] oder KBr oder NaBr ersetzt oder man unterwirft das durch Reduktion der $NO_2$-Gruppe erhaltene Anilin einer Sandmeyer-Reaktion.

Das erfindungsgemässe Verfahren zur Herstellung von Phenoxy-phenoxy-alkancarbonsäure Derivatender allgemeinen Formel I ist dadurch gekennzeichnet, dass man in an sich bekannter Weise einen Diphenyläther der allgemeinen Formel II

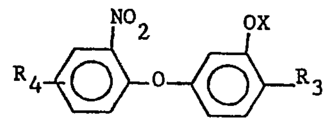

(II)

worin X Wasserstoff oder das Kation eines Alkali- oder Erdalkalimetalls bedeutet und $R_3$ und $R_4$ die in der allgemeinen Formel I an gegebene Bedeutung haben,
mit einem $\alpha$-Halogen-carbonsäurederivat der allgemeinen Formel III

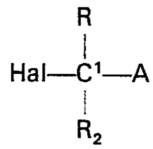

(III)

worin A, $R_1$ und $R_2$ die in der allgemeinen Formel I an gegebene Bedeutung haben, während Hal ein Halogenatom, vorzugsweise Chlor oder Brom bedeutet, umsetzt.

Die Reaktionspartner der allgemeinen Formeln II und III werden in etwa stöchiometrischen Mengen eingesetzt. Die Reaktion erfolgt vorteilhafterweise in Anwesenheit eines organischen, den Reaktionspartnern gegenüber inerten Lösungsmittels, bei einer Temperatur zwischen Raumtemperatur und der Siedetemperatur des Reaktionsgemisches, gegebenenfalls d.h. wenn X Wasserstoff bedeutet, in Gegenwart eines säurebindenden Mittels.

Nach Beendigung der Reaktion, welche zwischen 1 und 8 Stunden dauern kann, wird das Reaktionsprodukt isoliert, z.B. durch Abdestillieren des Lösungsmittels und Eingiessens des Rückstandes in Eiswasser.

Als Lösungsmittel kommen für diese Reaktion vor allem organische aprotische Lösungsmittel in Frage, welche die Reaktionspartner zwar lösen aber nicht mit ihnen reagieren. Die Kondensationsmittel sind anorganische Basen, wie NaOH, KOH, $NaOCH_3$ etc., aber auch organische Basen wie tertiäre Amine.

Die nachfolgenden Beispiele veranschaulichen erfindungsgemässe Herstellungsverfahren für einige Wirkstoffe der allgemeinen Formel I. Weitere in entsprechender Weise hergestellte Wirkstoffe sind in der anschliessenden Tabelle aufgeführt. Temperaturangaben bezeihen sich jeweils auf Celsius-Grade.

Die Phenoxy-phenoxy-alkan-carbonsäure-Derivate der allgemeinen Formel I sind stabile Verbindungen, welche in üblichen organischen Lösungsmitteln, wie Alkoholen, Aethern, Ketonen, Dimethylformamid, Dimethylsulfoxyd etc. löslich sind.

Beispiel 1

34,1 g (0,1 Mol) 2-Nitro-4-trifluormethyl-3'-acetoxy-diphenyläther werden in 200 ml Essigsäure gelöst und unter gutem Rühren bei 40 äquimolare Mengen Chlor eingeleitet. Die Reaktion wird gaschromatographisch verfolgt. Nach beendeter Reaktion giesst man die erhaltene Lösung in Eiswasser und extrahiert mit Toluol/Essigester 1:1. Nach Abdestillieren des organischen Lösungsmittels erhält man ein Oel, das mittels 30%iger methanolischer Natronlauge zum freien Phenol verseift wird. Das so erhaltene Oel hat einen Kp: von 159—165° bei 0,03 mm Hg. Ausbeute 23,5 g.

23,5 g 2-Nitro-4-trifluormethyl-3'-hydroxy-4'-chlor-diphenyläther werden in 30 ml Aethylmethylketon gelöst. Dazu setzt man 18 ml 2-Brom-propionsäuremethylester, 18 g Kaliumcarbonat und eine Spatelspitze Kaliumjodid. Das Reaktionsgemisch wird über Nacht bei 80° Badtemperatur gerührt, von unlöslichen Anteilen abfitlriert und das Lösungsmittel abdestilliert. Das so erhaltene Oel wird im Hochvakuum destilliert. Kp: 172°/0,015 mm Hg Ausbeute 20,8 g.

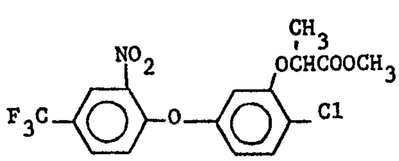

Beispiel 2

a) 68,2 g 2-Nitro-4-trifluormethyl-3'-acetoxy-diphenyläther werden in 80 ml Aethylenchlorid gelöst und bei 0—5° 40 ml Nitriersäure (1:1) zugetropft. Nach beendetem Zutropfen wird noch 1/2 Stunde ausgerührt, das Reaktionsprodukt in Wasser gegossen und das ausgefallene Produkt mit Essigester-Toluol 1:1 extrahiert. Nach Abdestillieren des organischen Lösungsmittels erhält man ein festes Produkt, das aus Alkohol/Wasser unkristallisiert wird. Smp.: 129—130°, Ausbeute: 47,4 g = 61,4% d.Th.

b) 47,4 g 2,4'-Dinitro-4-trifluormethyl-3'-acetoxydiphenyläther werden mit 200 ml Methanol versetzt und nach Zugabe der äquimolaren Menge methanolischer KOH eine halbe Stunde stehengelassen. Nach Abdestillieren des Lösungsmittels wird der Rückstand in Wasser gelöst und nach Ansäuern mit 2n HCl das ausgeschiedene Phenol mit Essigester/Toluol 1:1 extrahiert. Nach Abdestillieren des Lösungsmittel erhält man 44,5 g Oel, das beim stehen kristallisiert. Schmelzpunkt: 78—79°.

c) 17,2 g 2,4'-Dinitro-4-trifluormethyl-3'-hydroxydiphenyläther werden in 50 ml 2-Butanon gelöst und nach Zugabe von 13,4 g 2-Brom-propionsäuremethylester, 13,8 g Kaliumcarbonat und einer Spatelspitze Kaliumjodid über Nacht bei 80° Badtemperatur gerührt. Anschliessend wird filtriert, das Filtrat eingedampft und das erhaltene Oel im Rochvakuum destilliert. Kp: 217°/0,05 Torr Ausbeute: 6,2 g

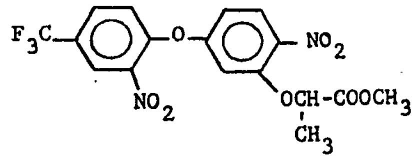

Auf analoge Weise zu diesen Beispielen sind folgende Verbindungen hergestellt worden:

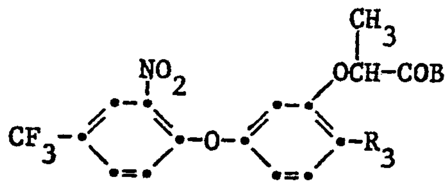

| No. | $R_3$ | B | phys. Kenndaten |
|---|---|---|---|
| 1 | Cl | $OCH_3$ | Kp 172°/0.15 mbar |
| 2 | Cl | $OC_2H_5$ | Kp 173°/0.04 mbar |
| 3 | Cl | $OC_2H_4Cl$ | Smp. 70–74° |
| 4 | Cl | $OC_4H_9$sec | $n_D^{24}$ 1.5115 |
| 5 | Cl | $OC_3H_7$n | Kp 190°/0.001 mbar |
| 6 | Cl | $OC_4H_9$iso | Smp. 80–81° |
| 7 | Cl | $OC_2H_4OCH_3$ | Kp 200°/0.001 mbar |
| 8 | Cl | OH | Smp. 168–170° |
| 9 | Cl | $O^{\ominus}$ $Na^{\oplus}$ | Smp. > 210° Z |
| 10 | Cl | $OCH_2{-}CH{=}CH_2$ | Kp 188°/0.35 mbar |
| 11 | Cl | 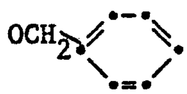 | Kp 235°/0.001 mbar |
| 12 | Cl | $OCH_2{-}C{\equiv}CH$ | $n_D^{26}$ 1.5313 |
| 13 | Cl | $OCH_2CH(CH_3)C_2H_5$ | $n_D^{25}$ 1.5091 |
| 14 | Cl | $OCH(CH_3)CH_2OCH_3$ | $n_D^{25}$ 1.5163 |
| 15 | Cl | 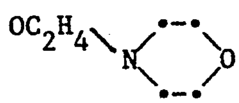 | $n_D^{22}$ 1.5267 |
| 16 | Cl | 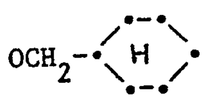 | Kp 195°/0.04 mbar |
| 17 | Cl | $OC_3H_7$ iso | Kp 185–7°/0.04 mbar |
| 18 | Cl | $OCH_2CF_3$ | $n_D^{26}$ 1.5020 |
| 19 | Cl | 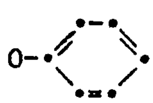 | Smp. 127–29° |
| 20 | Cl | $OC_2H_4CN$ | $n_D^{27}$ 1.5177 |
| 21 | Cl | $SCH_3$ | viskoses Oel |
| 22 | Cl | $SCH_2CH{=}CH_2$ | Kp. 200–230°/0.01 mbar |

| No. | $R_3$ | B | phys. Kenndaten |
|---|---|---|---|
| 23 | Cl | 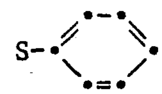 | Kp. 230°/0.01 mbar |
| 24 | Cl | $ON=C(CH_3)_2$ | $n_D^{25}$ 1.5372 |
| 25 | Cl | $NH_2$ | Smp > 180° Z |
| 26 | Cl | $NHC_2H_4OCH_3$ | $n_D^{25}$ 1.5250 |
| 27 | Cl | 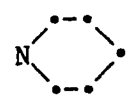 | $n_D^{26}$ 1.5420 |
| 28 | Cl | 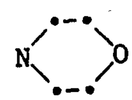 | viskoses Oel |
| 29 | Cl | $N(CH_3)OCH_3$ | Smp. 90–96° |
| 30 | Br | $OCH_3$ | Kp. 185°/0.03 mbar |
| 31 | I | $OCH_3$ | Kp. 190°/0.004 mbar |
| 32 | $NO_2$ | $OCH_3$ | Kp. 217°/0.05 mbar |
| 33 | $NO_2$ | $OCH_2-CH=CH_2$ | $n_D^{22}$ 1.5337 |
| 34 | $NO_2$ | $OCH_2-C\equiv CH$ | $n_D^{26}$ 1.5312 |
| 35 | $NO_2$ | $N(C_2H_5)_2$ | Kp. 190°/0.005 mbar |
| 36 | $NO_2$ | OH | Smp. 135–42° |
| 37 | CN | $OCH_3$ | $n_D^{22}$ 1.5431 |

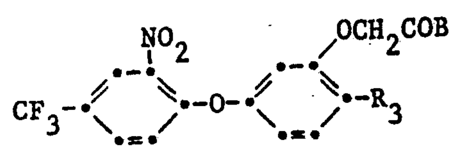

| No. | $R_3$ | B | phys. Kenndaten |
|---|---|---|---|
| 38 | $NO_2$ | OH | Smp. 98–104° |
| 39 | $NO_2$ | $OCH_3$ | Smp. 107–8° |
| 40 | Cl | $OCH_3$ | Smp. 108° |

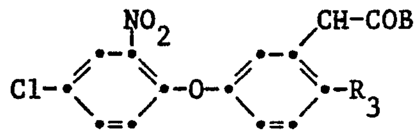

| No. | $R_3$ | B | phys. Kenndaten |
|---|---|---|---|
| 41 | Cl | $OCH_3$ | viskoses Oel |
| 42 | Cl | OH | Smp. 125° |
| 43 | Cl | $OC_2H_5$ | $n_D^{25}$ 1.5705 |
| 44 | Cl | $OC_3H_7$ iso | $n_D^{25}$ 1.5604 |
| 45 | Cl | $OC_4H_9$ iso | $n_D^{25}$ 1.5596 |
| 46 | Cl | $OC_2H_4N(C_2H_5)_2$ | viskoses Oel |
| 47 | Br | $OCH_3$ | Kp. 185°/0.04 mbar |
| 48 | $NO_2$ | $OCH_3$ | Smp. 87–88° |
| 49 | $NO_2$ | $SCH_3$ | viskoses Oel |

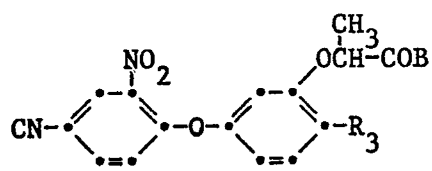

| No. | $R_3$ | B | phys. Kenndaten |
|---|---|---|---|
| 50 | Cl | $OCH_3$ | Kp. 169°/0.001 mbar |
| 51 | $NO_2$ | $OCH_3$ | Kp. 205°/0.005 mbar |

Die Erfindung betrifft auch herbizide und pflanzenwachstumsregulierende Mittel, welche einen neuen Wirkstoff der allgemeinen Formel I enthalten, sowie Verfahren zur pre- und post-emergenten Umkrautbekämpfung und zur Hemmung des Pflanzenwuchses von monocotylen und dicotylen Pflanzen, insbesondere Gräsern, Getreide, Soja und Tabakgeiztrieben.

Die erfindungsgemässen Mittel können in den überlichen Formulierungen vorliegen.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsgranulate, Imprägnierungs-granulate und Homogen-granulate; |
| in Wasser dispergierbare Wirkstoffkonzentrate: | Spritzpulver, (wettable powder), Pasten, Emulsionen; |
| flüssige Aufarbeitungsformen: | Lösungen. |

Der Gehalt an Wirkstoff in den oben beschriebenen Mitteln liegt zwischen 0,1 bis 95%, bevorzugt

zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Die Wirkstoffe der allgomeinen Formel I können beispielsweise wie folgt formuliert werden (Teile bedeuten Gewichtsteile):

Stäubemittel:

Zur Herstellung eines a) 5%igen und b) 2%igen Stäubemittels werden die folgenden Stoffe verwendet:

a) 5 Teile 2-Chlor-5-(2'-Nitro-4'-trifluormethylphenoxy)-α-phenoxy-propionsäure-methyl-ester

95 Teile Talkum

b) 2 Teile des obigen Wirkstoffes

1 Teil hochdisperse Kieselsäure

97 Teile Talkum;

Die Wirkstoffe werden mit den Trägerstoffen vermischt und vermahlen.

Granulate

Zur Herstellung eines 5%igen Granulates werden die folgenden Stoffe verwendet:

5 Teile des obigen Wirkstoffes

0,25 Teile Epichlorhydrin

0,25 Teile Cetylpoläthylenglykoläther mit 8 Mol Aethylenoxid,

3,50 Teile Polyäthylenglykol,

91 Teile Kaolin (Korngrösse 0,3 bis 0,8 mm)

Die Aktivsubstanz wird mit Epichlorhydrin vermischt und in 6 Teilen Aceton gelöst, hierauf werden Polyäthylenglycol und Cetylpolyäthylenglycoläther zugesetzt. Die so erhaltene Lösung wird auf Kaolin aufgesprüht und anschliessend im Vakuum verdampft.

Spritzpulver

Zur Herstellung eines a) 50%igen, b) 25%igen und c) 10%igen Spritzpulvers werden folgende Bestandteile verwendet:

a) 50 Teile 2 - Chlor - 5 - (2' - nitro - 4' - trifluormethyl - phenoxy) - α - phenoxy-propionsäure - methylester,

5 Teile Natriumdibutylnaphthylsulfonat,

3 Teile Naphthalinsulfonsäuren - Phenonolsulfonsäuren - Formaldehyd - Kondensat 3:2:1,

20 Teile Kaolin,

22 Teile Champagne-Kreide;

b) 25 Teile des obigen Wirkstoffes,

5 Teile Oleylmethyltaurid-Natrium-Salz,

2,5 Teile Naphthalinsulfonsäuren-Formaldehyd-Kondensat,

0,5 Teile Carboxymethylcellulose,

5 Teile neutrales Kalium-Aluminium-Silikat,

62 Teile Kaolin,

c) 10 Teile des obigen Wirkstoffs,

3 Teile Gemisch der Natriumsalze von gesättigten Fettalkoholen,

5 Teile Naphthalinsulfonsäuren-Formaldehyd-Kondensat,

82 Teile Kaolin.

Der angegebene Wirkstoff wird auf die entsprechenden Trägerstoffe aufgezogen und anschliessend vermischt und vermahlen. Man erhält so Spritzpulver von vorzüglicher Benetzbarkeit und Schwebefähigkeit, die durch Verdünnen mit Wasser Suspensionen jeder gewünschten Wirkstoffkonzentration ergeben.

Paste

Zur Herstellung einer 45%igen Paste werden folgende Stoffe verwendet:

45 Teile 2 - Chlor - 5 - (2' - nitro - 4' - trifluormethyl - phenoxy) - $\alpha$ - phenoxy-propionsäure - methylester,

5 Teile Natriumaluminiumsilikat,

14 Teile Cetylpolyäthylenglykoläther mit 8 Mol Aethylenoxid,

1 Teile Oleylpolyäthylenglykoläther mit 5 Mol Aethylenoxid,

2 Teile Spindelöl,

23 Teile Wasser,

10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen. Die Suspension eignen sich zur Behandlung von Rasenanlagen.

Emulsionskonzentrat

Zur Herstellung eines 25%igen Emulsionskonzentrates werden

25 Teile 2 - Chlor - 5 - (2' - nitro - 4' - trifluormethylphenoxy) - $\alpha$ - phenoxy - propionsäure - methylester,

5 Teile Mischung von Nonylphenolpolyoxyäthylen und Calcium-dodecylbenzolsulfonat,

35 Teile 3,5,5-Trimethyl-2-cyclohexen-1-on,

35 Teile Dimethylformamid

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere der von der allgermeinen Formel I umfassten Verbindungen verwendet werden.

Die in den erfindungsgemässen Mitteln enthaltenen Wirkstoffe beeinflussen das Pflanzenwachstum in verschiedener Weise. So hemmen, verzögern oder unterbinden sie in erster Linie das Wachstum und die Keimung. Es handelt sich dabei also sowohl um pre- und postemergente Herbizidwirkung als auch um Wuchshemmung.

Erfindungsgemässe Mittel, die als aktive Komponente mindestens eine Verbindung der allgemeinen Formel I enthalten, eignen sich besonders zur Hemmung und Kontrolle des Pflanzenwachstums von monocotylen und dicotylen Pflanzen, wie Gräsern, Sträuchern, Bäumen, Getreide- und Leguminosenkulturen, Zuckerrohr, Tabak, Soja, Zwiebeln- und Kartoffelknollen, Zierpflanzen, Obstbäumen und Reben.

Die von den neuen Wirkstoffen der allgemeinen Formel I in erster Linie erzielte Wirkung besteht in der gewünschten Reduktion der Pflanzengrösse, insbesondere der Wuchshöhe. Im allgemeinen ist damit eine gewisse Aenderung der Pflanzenform verbunden. In unmittelbarem Zusammenhang zur Verminderung der Wuchshöhe erfährt die Pflanze eine Festigung. Blätter und Stengel sind kräftiger ausgebildet. Durch Verkürzung der Internodienabstände an monocotylen Pflanzen wird die Knickfestigkeit erhöht. Auf diese Weise können Ernteausfälle durch Gewittersturm, Daurregen, usw., die normalerweise zu einem Lagern von Getreide- und Leguminosenkulturen führen, weitgehend verhindert und damit die Erntearbeit erleichtert werden. Als Nebeneffekt führt verminderte Wuchshöhe bei Nutzpflanzen zu einer Einsparung an Düngemitteln.

Eines der wichtigsten Probleme an reinen Grasbepflanzungen ist jedoch der Grasschnitt selbst, sie es an Grünanlagen in Wohngegenden, auf Industriegeländen, auf Sportplätzen, an Autostrassen, Flugpisten, Eisenbahndämmen oder Uferböschungen von Gewässern. In all diesen Fällen ist ein periodisches Schneiden des Rasens bzw. des Graswuchses notwendig. Dies ist nicht nur im Hinblick auf Arbeitskräfte und Maschinen sehr aufwendig, sondern bringt im Verkehrsbereich auch erhebliche Gefahren für das betroffene Personal und die Verkehrsteilnehmer mit sich.

Es besteht daher gerade in Gebieten mit grossen Verkehrsnetzen ein dringendes Bedürfnis, die im Hinblick auf die Verfestigung von Seitenstreifen und Böschungen and Verkehreswegen notwendige Grasnarbe einersiets zu erhalten und zu pflegen, andererseits aber mit einfachen Massnahmen während der gesamten Vegetationsperiode auf einer mittleren Wuchshöhe zu halten. Dies wird durch Applikation erfindungsgemässer Wirkstoffe der allgemeinen Formel I auf sehr günstige Weise erreicht.

In analoger Weise kann durch Behandlung von Bäumen, Sträuchern und Hecken, vor allem in Wohnund Industriegebeiten, mit erfindungsgemässen Verbindungen der allgemeinen Formel I die arbeitsaufwendige Schnittarbeit reduziert werden.

Durch den Einsatz erfindungsgemässer Wirkstoffe der allgemeinen Formel I können auch das Triebwachstum und/oder die Fruchtbarkeit von Obstbäumen und Reben vorteilhaft beeinflusst werden.

Die Wirkstoffe der allgemeinen Formel I finden auch Anwendung zur Hemmung des Wachstums unerwünschter Geiztriebe, z.B. bei Tabak und Zierpflanzen, wodurch das arbeitsintensive Ausbrechen dieser Triebe von Hand vermieden wird, ferner zur Austriebhemmung bei lagernden Knollen, beispielsweise bei Zierpflanzenknollen, bei Zwiebeln und Kartoffeln, und schliesslich zur Ertragssteigerung bei stark vegetativ wachsenden Kulturpflanzen, wie Soja und Zuckerrohr, indem durch Applikation erfindungsgemässer Wirkstoffe der Uebergang von der vegetativen zur generativen Wachstumsphase beschleunigt wird.

Bevorzugt setzt man die erfindungsgemässen Wirkstoffe der allgemeinen Formel I zur Wachstumshemmung an Gräsern, Getreidekulturen, Tabak, Soja und Zierpflanzen ein.

Die Aufwandmengen sind verschieden und vom Applikationszeitpunkt abhängig. Sie liegen im allgemeinen zwischen 0,1 und 5 kg Wirkstoff pro Hektar, bei Applikation vor dem Auflaufen der Pflanzen und für die Behandlung von bestehenden Kulturen vorzugsweise bis zu 4 kg pro Hektar.

Ferner eignen sich viele der Wirkstoffe der allgemeinen Formel I und diese enthaltende Mittel auch für andere Beeinflussungen des Pflanzenwachstums, wie insbesondere zur Erleichterung der Frucht- und Blattabszission durch Ausbildung von Trenngeweben an den Frucht- und Blattstielen, was eine beträchtliche Reduktion der Abreisskraft und somit eine grosse Ernteerleichterung mit sich bringt.

Grosse wirtschaftliche Bedeutung hat die Erleichterung der Fruchtabszission bei der mechanischen und manuellen Ernte von Oliven und Citrusfrüchten gewonnen. Blattabszissionswirkung und Defoliation ist bei der Baumwollernte von Bedeutung.

Die Entfaltung der Wirkung der erfindungsgemässen Wirkstoffe erfolgt sowohl über die oberirdischen Pflanzenteile (Kontakwirkung), insbesondere die Blätter, als auch über den Boden, als preemergentes Herbizid (Keimhemmung).

Die Wirkung als starke Wachstumshemmer zeigt sich darin, dass die meisten post-emergent behandelten Pflanzenarten nach dreiwöchiger Versuchsdauer einen Wachstumsstillstand zeigen, wobei die behandelten Pflanzenteile eine dunkelgrüne Färbung annehmen. Die Blätter fallen aber nicht ab.

Diese Wuchshemmung tritt bei einigen Pflanzenarten schon bei einer Dosierung von 0,5 kg/ha und darunter auf.

Da nicht alle Pflanzenarten gleich stark gehemmt werden, ist bei Wahl einer bestimmten niederen Dosierung ein selektiver Einsatz möglich.

Die erfindungsgemässen Wirkstoffe sind auch interessante Kombinationspartner für eine Reihe von Herbiziden der Phenylharnstoff- und Triazinreihe in Getreidekulturen, Mais, Zuckerrohr bezw. im Obst- und Weinbau.

Dabei wird die Unkrautdecke nicht beseitigt, sondern nur so stark gehemmt, dass keine Konkurrenzierung der Kulturpflanzen mehr auftritt.

Die neuen Wirkstoffe der allgemeinen Formel I zeichnen sich überdies durch eine sehr starke preemergente Herbizidwirkung aus, sind also auch ausgeprägte Keimungshemmer.

Zum Nachweis der Brauchbarkeit als Herbizide (pre- und post-emergent) und als Wuchshemmer dienten folgende Testmethoden:

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Suspension der Wirkstoffe, erhalten aus einem 25%-igen Spritzpulver, behandelt. Es wurden vier verschiedene Konzentrationsreihen angewendet, entsprechend

4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben

2—8 = Zwischenstufen der Schädigung

9 = Pflanzen ungeschädigt (wie unbehandelte Kontrolle).

Post-emergente Herbizid-Wirkung (Kontaktherbizid)

Eine grössere Anzahl (mindestens 7) Unkräuter und Kulturpflanzen, sowohl monocotyle wie dicotyle, wurden nach dem Auflaufen (im 4-bis-6-Blattstadium) mit einer wässrigen Wirkstoffemulsion in Dosierungen von 0,5; 1; 2 und 4 kg Wirksubstanz pro Hektar auf die Pflanzen gespritzt und diese bei 24—26°C und 45—60% rel. Luftfeuchtigkeit gehalten. 5 Tage und 15 Tage nach Behandlung wird der Versuch ausgewertet und das Ergebnis wie im pre-emergent-Versuch nach derselben Notenskala bonitiert.

Die geprüften Verbindungen gemäss der Erfindung zeigten auf einigen Pflanzen ausgeprägte kontaktherbizide Wirkung und auf vielen Pflanzen Wachstumsstillstand als Symptom der wachstumshemmenden Eigenschaften.

Wuchshemmung bei Gräsern

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der allgemeinen Formel I bespritzt. Die Wirkstoffmenge betrug umgerechnet 5 kg Aktivsubstanz pro Hektar. 10 und 21 Tage nach Applikation wurde das Wachstum der Gräser beurteilt.

Wuchshemmung bei Getreide

In Kunststoffbechern wurde Sommerweizen (Triticum aestivum), Sommergerste (Hordeum vulgare) und Roggen (Secale) in sterilisierter Erde angesät und im Gewächshaus gezogen. D'e Getreidesprösslinge werden 5 Tage nach Aussaat mit einer Spritzbrühe des Wirkstoffs behandelt. Die Blattapplikation entsprach 6 kg Wirkstoff pro Hektar. Die Auswertung erfolgt nach 21 Tage.

**Patentansprüche**

1. 3-(2'-Nitro-phenoxy)-$\alpha$-phenoxy-alkancarbonsäure derivate der allgemeinen Formel I

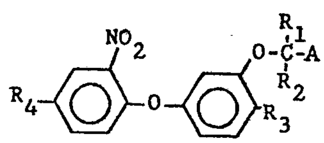

(I)

worin $R_1$ Wasserstoff, $C_1$—$C_4$-Alkyl oder $C_2$—$C_8$-Alkoxyalkyl,
$R_2$ Wasserstoff oder $C_1$—$C_4$-Alkyl,
$R_3$ Halogen, Cyano oder Nitro,
$R_4$ Halogen, Cyano oder Trifluormethyl,
A Cyan oder die Gruppe —COB,
B einen Rest $OR_5$, $SR_6$ oder $NR_7R_8$,
$R_5$ Wasserstoff oder

das Kation einer Base $\frac{1}{n} M^{n\oplus}$,

wobei

M ein Alkali-, Erdalkalimetall-Kation oder einen Ammoniumrest

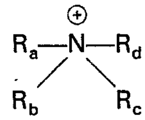

n als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, —$NH_2$ oder $C_1$—$C_4$-Alkoxy substituierten $C_1$—$C_4$-Alkylrest bedeuten,

$R_5$ bedeutet weiter einen $C_1$—$C_8$-Alkylrest, der unsubstituiert oder durch Halogen, Cyan, Nitro, Hydroxyl oder einen 5—6 gliedrigen Stickstoff enthaltenden Heterocyclus substituiert sein kann sowie einen $C_2$—$C_8$-Alkylrest, der durch Sauerstoff, Schwefel, Stickstoff, Carbonyl oder Carbonyloxy unterbrochen sein kann, einen $C_3$—$C_6$-Cycloalkylrest, einen $C_3$—$C_6$-Alkenyl- oder Alkinylrest, der durch Halogen substituiert sein kann, einen Phenyl oder Benzylrest der durch Halogen, Cyan, Nitro, $C_1$—$C_4$-Alkyl, $C_1$—$C_4$-Alkoxy oder Trifluormethyl substituiert sein kann,

$R_6$ bedeutet einen $C_1$—$C_8$-Alkylrest der durch Halogen, Cyano, Nitro oder Hydroxyl substituiert, oder durch Sauerstoff, Schwefel, Stickstoff, Carbonyl oder Carbonyloxy unterbrochen sein kann, einen $C_3$—$C_6$-Cycloalkylrest, einen $C_3$—$C_6$-Alkenyl- oder Alkinylrest, der durch Halogen substituiert sein kann, einen Phenyl- oder Benzylrest, der durch Halogen, Cyan, Nitro, $C_1$—$C_4$-Alkyl, $C_1$—$C_4$-Alkoxy oder Trifluormethyl substituiert sein kann,

$R_7$ Wasserstoff, einen $C_1$—$C_8$-Alkylrest, der durch Halogen, Cyan oder Hydroxyl substituiert sein kann, sowie einen $C_2$—$C_8$-Alkylrest, der durch Sauerstoff unterbrochen sein kann,

$R_8$ Wasserstoff, einen $C_1$—$C_4$-Alkylrest oder einen $C_3$—$C_4$-Alkylrest,

$R_7$ und $R_8$ zusammen mit dem Stickstoffatom an das sie gebunden sind, auch einen 5—6 gliedrigen, heterocyclischen Rest, der durch Sauerstoff, Schwefel, eine Imino-$C_1$—$C_4$-Alkylimino- oder Phenyliminogruppe unterbrochen sein kann, bedeuten.

2. Die Verbindungen gemäß Anspruch 1, in denen $R_1$ Wasserstoff bedeutet, während $R_2$, $R_3$, $R_4$ und A die gegebene Bedeutung haben.

3. Die Verbindungen gemäß Anspruch 1, in denen $R_1$ Wasserstoff, A —COB und B —$COR_5$ bedeutet während $R_2$, $R_3$, $R_4$ die an gegebene Bedeutung haben.

4. Die Verbindungen gemäß Anspruch 1, in denen $R_1$ Wasserstoff, A —CDB und B —$NR_7R_8$ bedeuten, während $R_2$, $R_3$ und $R_4$ die an gegebene Bedeutung haben.

5. Die Verbindungen gemäß Anspruch 1, in denen $R_1$ Wasserstoff, A —COB und B —$SR_6$ bedeuten, während $R_2$, $R_3$ und $R_4$ die an gegebene Bedeutung haben.

6. 2 - Chlor - 5 - (2′ - nitro - 4′ - trifluormethyl - phenoxy) - $\alpha$ - phenoxy - propionsäure - methylester.

7. Verfahren zur Herstellung der 3-(2′-Nitro-phenoxy)-$\alpha$-phenoxy-alkancarbonsäure-Derivate, Anspruch 1, dadurch gekennzeichnet, dass in an sich bekannter Weise ein Diphenyläther der allgemeinen Formel II

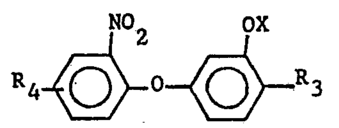

(II)

worin $R_3$ und $R_4$ die an gegebene Bedeutung haben und X Wasserstoff oder das Kation eines Alkalioder Erdalkalimetalles bedeutet, mit einem $\alpha$-Halogencarbonsäurederivat der allgemeinen Formel III

$$\begin{array}{c} R_1 \\ | \\ \text{Hal—C—A} \\ | \\ R_2 \end{array} \qquad \text{(III)}$$

worin A, $R_1$ und $R_2$ die an gegebene Bedeutung haben und Hal ein Halogenatom bedeutet, umsetzt.

8. Herbizides und auf das Pflanzenwachstum einwirkendes Mittel, dadurch gekennzeichnet, dass es als Wirkstoff mindestens eine Verbindung der allgemeinen Formel I, Anspruch 1, enthält.

9. Die Verwendung der 3-(2′-Nitro-phenoxy)-$\alpha$-phenoxy-alkancarbonsäure-Derivate der allgemeinen Formel I, Anspruch 1, oder sie enthaltender Mittel zur Unkrautbekämpfung.

10. Die Verwendung der 3-(2′-Nitro-phenoxy)-$\alpha$-phenoxy-alkancarbonsäure-Derivate der allgemeinen Formel I, Anspruch 1 oder sie enthaltender Mittel zur Regulierung des Pflanzenwachstums.

**Revendications**

1. Dérivés d'acide 3-(2-nitro-phénoxy)-alpha-phénoxy-alcanecarboxylique de formule générale I

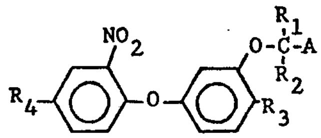

(I)

où

$R^1$ représente un hydrogène, un alcoyle en $C_1$ à $C_4$ ou un alcoxyalcoyle en $C_2$ à $C_8$
$R_2$ représente un hydrogène ou un alcoyle en $C_1$ à $C_4$
$R_3$ un halogène, un cyano ou nitro
$R_4$ un halogène, un cyano ou un trifluorométhyle
A un cyano ou le groupe —COB
B un radical $OR_5$, $SR_6$ ou $NR_7R_8$
$R_5$ un hydrogène ou le cation d'une base

$$\frac{1}{n} M^{n\oplus}$$

où

M représente un cation de métal alcalin ou alcalinoterreux ou un radical ammonium

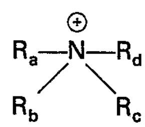

n le nombre entier 1, 2 ou 3 selon la valence du cation, cependant que $R_a$, $R_b$, $R_c$ et $R_d$ représentent indépendamment l'un de l'autre un hydrogène, un benzyle ou un radical alcoyle en $C_1$ à $C_4$ éventuellement substitué par —OH, —$NH_2$ ou un alcoxy en $C_1$ à $C_4$,

$R_5$ représente en outre un radical alcoyle en $C_1$ à $C_8$, qui peut être non substitué ou substitué par un halogène, un cyano, un nitro, un hydroxyle ou un hétérocycle à 5 à 6 chaînons contenant de l'azote, ainsi qu'un radical alcoyle en $C_2$ à $C_8$, qui peut être interrompu par un oxygène, un soufre, un azote, un carbonyle ou un carbonyloxy, un radical cycloalcoyle en $C_3$ à $C_8$, un radical alcényle ou alcynyle en $C_3$ à $C_6$, qui peut être substitué par un halogène, un radical phényle ou benzyle, qui peut être substitué par un halogène, un cyano, un nitro, un alcoyle en $C_1$ à $C_4$, un alcoxy en $C_1$ à $C_4$ ou un trifluorométhyle,

$R_6$ représente un radical alcoyle en $C_1$ à $C_8$, qui peut être substitué par un halogène, un cyano, un nitro ou un hydroxy, ou être interrompu par un oxygène, un soufre, un azote, un carbonyle ou un carbonyloxy, un radical cycloalcoyle en $C_1$ à $C_6$,
un radical alcényle ou alcynyle en $C_3$ à $C_6$, qui peut être substitué par un halogène,
un radical phényle ou benzyle, qui peut être substitué par un halogène, un cyano, un nitro, un alcoyle en $C_1$ à $C_4$, un alcoxy en $C_1$ à $C_4$ ou un trifluorométhyle,

$R_7$ représente un hydrogéne, un radical alcoyle en $C_1$ à $C_8$, qui peut être substitué par un halogène, un cyano ou un hydroxyle, ainsi qu'un radical alcoyle en $C_2$ à $C_8$, qui peut être interrompu par un oxygène,

$R_8$ un hydrogène, un radical alcoyle en $C_1$ à $C_4$ ou un radical alcoyle en $C_3$ à $C_4$,

$R_7$ et $R_8$ représentent également ensemble avec l'atome d'azote auquel ils sont liés, un radical hétérocyclique à 5 à 6 chaînons, qui peut être interrompu par un oxygène, un soufre, un groupe imino, alcoylimino en $C_1$ à $C_4$ ou phénylimino.

2. Les composés selon la revendication 1 où $R_1$ représente un hydrogène, tandis que $R_2$, $R_3$, $R_4$ et A ont la signification donnée.

3. Les composés selon la revendication 1, où $R_1$ représente un hydrogène, A —COB et B —$COR_5$, tandis que $R_2$, $R_3$ et $R_4$ ont la signification donnée.

4. Les composés selon la revendication 1, où $R_1$ représente un hydrogène, A —COB et B —$NR_7R_8$ tandis que $R_2$, $R_3$ et $R_4$ ont la signification donnée.

5. Les composés selon la revendication 1 où $R_1$ représente un hydrogène, A —COB et B —$SR_6$ tandis que $R_2$, $R_3$ et $R_4$ ont la signification donnée.

6. L'ester méthylique de l'acide 2-chloro-5-(2'-nitro-4'-trifluorométhyl-phénoxy)-alpha-phénoxy-propionique.

7. Procédé de préparation de dérivés d'acide 3-(2'-nitro-phénoxy)alpha-alcanecarboxylique selon la revendication 1, caractérisé en ce qu'on fait réagir de façon classique un diphényléther de formule générale II

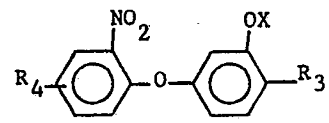

(II)

où $R_3$ et $R_4$ ont la signification donnée et X représente un hydrogène ou le cation d'un métal alcalin ou alcalino-terreux, avec un dérivé d'acide alpha-halogènecarboxylique de formule générale III

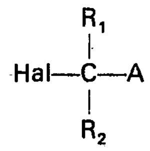

(III)

où A, $R_1$ et $R_2$ ont la signification donnée et où Hal représente un atome d'halogène.

8. Agent herbicide et agissant sur la croissance des plantes, caractérisé en ce qu'il contient comme matière active au moins un composé de formule générale I de la revendication 1.

9. Application des dérivés d'acide 3-(2'-nitrophénoxy)-alpha-phénoxy-alcanecarboxylique de formule générale I de la revendication I ou d'agents qui les contiennent à la lutte contre les mauvaises herbes.

10. Application des dérivés d'acide 3-(2'-nitrophénoxy)-alpha-phénoxy-alcanecarboxylique de formule générale I de la revendication 1 ou d'agents qui les contiennent à la régulation de la croissance des plantes.

**Claims**

1. A 3-(2'-nitrophenoxy)-$\alpha$-phenoxyalkanecarboxylic acid derivative of the general formula I

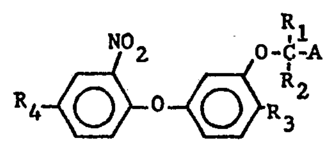

(I)

wherein
$R_1$ represents hydrogen, $C_1$—$C_4$alkyl or $C_2$—$C_8$alkoxyalkyl,
$R_2$ represents hydrogen or $C_1$—$C_4$alkyl,
$R_3$ represents halogen, cyano or nitro,
$R_4$ represents halogen, cyano or trifluoromethyl,
A represents cyano or the group —COB,
B represents a radical $OR_5$, $SR_6$ or $NR_7R_8$,
$R_5$ represents hydrogen or the cation of a base

$$\frac{1}{n} M^{n\oplus},$$

wherein
M represents an alkali metal or alkaline earth metal cation or an ammonium radical

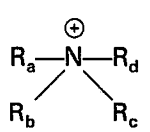

n as integer 1, 2 or 3 corresponds to the valency of the cation, whilst $R_a$, $R_b$, $R_c$ and $R_d$, each independently of the other, represent hydrogen, benzyl or an alkyl radical which is unsubstituted or substituted by —OH, —$NH_2$ or $C_1$—$C_4$alkoxy; and $R_5$ further represents,
a $C_1$—$C_8$alkyl radical which can be unsubstituted or substituted by halogen, cyano, nitro, hydroxyl or a 5- to 6-membered nitrogen-containing heterocyclic ring system, and a $C_2$—$C_8$alkyl radical which can be interrupted by oxygen, sulphur, nitrogen, carbonyl or carbonyloxy,
a $C_3$—$C_6$cycloalkyl radical,
a $C_3$—$C_6$alkenyl or alkynyl radical which can be substituted by halogen,

a phenyl or benzyl radical which can be substituted by halogen, cyano, nitro, $C_1$—$C_4$alkyl, $C_1$—$C_4$alkoxy or trifluoromethyl,

$R_6$ represents a $C_1$—$C_8$alkyl radical which can be substituted by halogen, cyano, nitro or hydroxyl or interrupted by oxygen, sulphur, nitrogen, carbonyl or carbonyloxy,

a $C_3$—$C_6$cycloalkyl radical,

a $C_3$—$C_6$alkenyl or alkynyl radical which can be substituted by halogen,

a phenyl or benzyl radical which can be substituted by halogen, cyano, nitro, $C_1$—$C_4$alkyl, $C_1$—$C_4$alkoxy or trifluoromethyl,

$R_7$ represents hydrogen, a $C_1$—$C_8$alkyl radical which can be substituted by halogen, cyano or hydroxyl, and a $C_2$—$C_8$alkyl radical which can be interrupted by oxygen,

$R_8$ represents a $C_1$—$C_4$alkyl radical or a $C_3$—$C_4$alkyl radical, and

$R_7$ and $R_8$, together with the nitrogen atom to which they are attached, also represent a 5- to 6-membered heterocyclic radical which can be interrupted by oxygen, sulphur, an imino, $C_1$—$C_4$alkylimino or phenylimino group.

2. A compound according to claim 1, wherein $R_1$ represents hydrogen and A, $R_2$, $R_3$ and $R_4$ have the given meanings.

3. A compound according to claim 1, wherein A represents —COB, B represents —$OR_5$, and $R_1$ represents hydrogen, and $R_2$, $R_3$, $R_4$ and $R_5$ have the given meanings.

4. A compound according to claim 1, wherein A represents —COB, B represents —$NR_7R_8$ and $R_1$ represents hydrogen, and $R_2$, $R_3$, $R_4$, $R_7$ and $R_8$ have the given meanings. .

5. A compound according to claim 1, wherein A represents —COB, B represents —$SR_6$ and $R_1$ represents hydrogen, and $R_2$, $R_3$ and $R_4$ have the given meanings.

6. α-[2-Chloro-5-(2′-nitro-4′-trifluoromethyl(phenoxy)phenoxy]propionic acid methyl ester.

7. A process for the manufacture of a 3-(2′-nitrophenoxy)-α-phenoxyalkanecarboxylic acid derivative according to claim 1, characterised in that a diphenyl ether of the general formula II

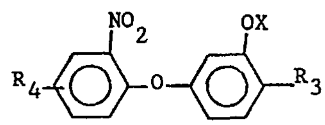

(II)

wherein $R_3$ and $R_4$ have the given meanings and X represents hydrogen or the cation of an alkali metal or alkaline earth metal, is reacted, in a manner known per se, with an α-halocarboxylic acid derivative of the general formula III

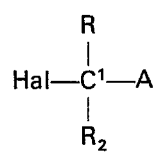

(III)

wherein A, $R_1$ and $R_2$ have the given meanings and Hal represents a halogen atom.

8. A herbicidal and plant growth-regulating composition, characterised in that it contains, as active ingredient, at least one compound of the general formula I in claim 1.

9. The use of a 3-(2′-nitrophenoxy)-α-phenoxyalkanecarboxylic acid derivative of the general formula I in claim 1, or of a composition containing such a compound, for controlling weeds.

10. The use of a 3-(2′-nitrophenoxy)-α-phenoxyalkanecarboxylic acid derivative of the formula I in claim 1, or of a composition containing such a compound, for regulating plant growth.